# EUROPÄISCHE PATENTANMELDUNG

(11) **EP 0 760 367 A2**
(43) Veröffentlichungstag der Anmeldung: **05.03.1997**
(21) Anmeldenummer: 96110273.8
(22) Anmeldetag: 26.06.1996
(51) Int. Cl.: C07D 211/58

(54) **Verfahren zur Herstellung von 4-Acylamino-2,2,6,6-tetramethylpiperidinen**

(30) Priorität: 01.09.1995 DE 19532215
(71) Anmelder: HÜLS AKTIENGESELLSCHAFT, 45764 Marl (DE)
(72) Erfinder: Kaufhold, Manfred, Dr., 45770 Marl (DE)

(57) **Zusammenfassung**

Das vorliegende Verfahren betrifft die Herstellung von 4-Acylamino-2,2,6,6-tetramethylpiperidinen aus 4-Amino-2,2,6,6-tetramethylpiperidin und Säureanhydriden. Bei diesem Verfahren wird die Umsetzung in Gegenwart von Carbonsäuren durchgeführt, worauf die Carbonsäure aus dem zunächst gebildeten Carbonsäuresalz des 4-Acylamino-2,2,6,6-tetramethylpiperidins mit Hilfe von Wasser bei erhöhter Temperatur abgespalten wird.

Bei diesem Verfahren fallen keine salzartigen Nebenprodukte an.

## Beschreibung

Die Erfindung betrifft ein neues Verfahren zur Herstellung von 4-Acylamino-2,2,6,6-tetramethylpiperidinen aus 4-Amino-2,2,6,6-tetramethylpiperidin und Säureanhydriden.

4-Acylamino-2,2,6,6-tetramethylpiperidine werden als Fungizide verwendet. Sie sind darüber hinaus wichtige Zwischenprodukte zur Herstellung der entsprechenden N-Oxyle, die als Stabilisatoren für olefinisch ungesättigte Verbindungen, wie beispielsweise Styrol oder Acrylsäure, eingesetzt werden.

Die Herstellung von Amiden des 4-Amino-2,2,6,6-tetramethylpiperidins, das auch Triacetondiamin (TAD) genannt wird, ist literaturbekannt. So beschreibt Harries in den Annalen, Band 417 (1918), auf Seite 120 die Herstellung des Essigsäuresalzes von Acetaminotetramethylpiperidin durch "Behandeln von Aminopiperidin mit Essigsäureanhydrid". In einer zweiten Stufe wird das Essigsäuresalz in Wasser gelöst und mit Natronlauge neutralisiert, wobei das Acetaminotetramethylpiperidin kristallin ausfällt. Bei diesem zweistufigen Verfahren fallen große, äquimolare Mengen an Natriumacetat an, die bei einer technischen Produktion entsorgt werden müßten.

In EP-A-0 387 489 wird vorgeschlagen, TAD mit einem Säurechlorid umzusetzen. Dabei fällt Kochsalz, dessen Entsorgung technisch aufwendig und daher kostspielig ist, in äquimolaren Mengen an. Beim Arbeiten mit Säurechloriden ist ein salzartiger Zwangsanfall nicht zu vermeiden.

Aufgabe der vorliegenden Erfindung war es, ein Verfahren zur Herstellung von 4-Acylamino-2,2,6,6-tetramethylpiperidinen aus TAD und Säureanhydriden bereitzustellen, bei dem keine salzartigen Abfälle anfallen.

Die Aufgabe wird überraschend dadurch gelöst, daß man die Umsetzung in Gegenwart von Carbonsäuren durchführt und die Carbonsäure aus dem zunächst hergestellten Carbonsäuresalz des 4-Acylamino-2,2,6,6-tetramethylpiperidins mit Hilfe von Wasser bei erhöhter Temperatur abspaltet.

Die Umsetzung kann durch folgendes Reaktionsschema beschrieben werden:

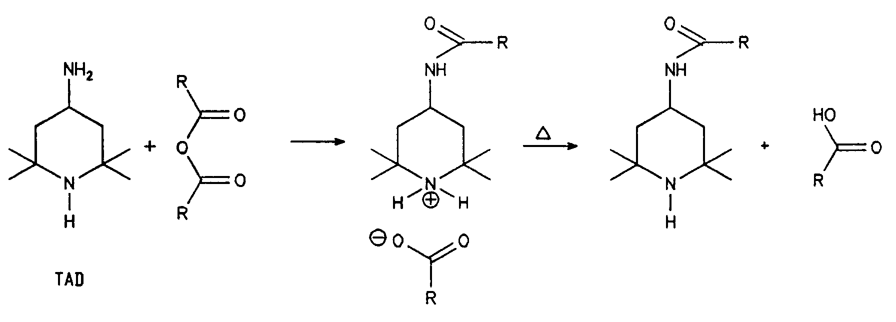

Die Substituenten R stehen dabei vorzugsweise für Alkyl mit 1 bis 7 C-Atomen sowie für Phenyl.

Beispiele für Alkylsubstituenten sind Methyl, Ethyl, n-Propyl, iso-Propyl, n-Butyl, iso-Butyl, Pentyl, Hexyl oder iso-Heptyl. Dabei sind im allgemeinen beide Substituenten R im Anhydrid gleich, obwohl auch gemischte Anhydride einsetzbar sind. Vorzugsweise weist auch die zugesetzte Carbonsäure den gleichen Rest R wie das Säureanhydrid auf.

Besonders bevorzugt stellt R einen Alkylrest mit 1 bis 3 C-Atomen dar.

Die Umsetzung wird vorzugsweise bei 80 bis 160 °C und die Spaltung unter Wasserzusatz bei 170 bis 250 °C durchgeführt. Ganz besonders bevorzugt stellt man eine Reaktionstemperatur von 90 bis 120 °C ein und spaltet und destilliert die Säure bei 190 bis 210 °C ab.

Es ist überraschend, daß die Spaltung der zunächst anfallenden salzartigen Verbindung mit Hilfe von Wasser bereits bei ca. 150 °C mit gutem Erfolg durchgeführt werden kann. Die erfindungsgemäße thermische Spaltung unter Wasserzusatz ist eine besonders schonende Methode. Dabei destilliert man ein Gemisch aus Wasser und Carbonsäure ab. Das Ende der Salzspaltung ist erreicht, wenn das Destillat keine Säure mehr enthält.

In einigen Fällen ist die Abspaltung der salzartig gebundenen Carbonsäure mit einer starken Sublimation verbunden. In diesen Fällen ist ein zusätzliches Lösemittel, das höher siedet als die abzuspaltende Säure, von Vorteil. So unterdrückt ein derartiges zusätzliches Lösemittel beispielsweise eine Sublimation bei der mit Wasser beschleunigten Essigsäureabspaltung bei der Herstellung von Acetyl-TAD. Man stellt dabei die Temperatur so ein, daß das Lösemittel im Reaktorsumpf siedet und an den oberen, kälteren Stellen des Reaktors wieder kondensiert. Dadurch wird das Festsetzen von Sublimat verhindert, während gleichzeitig ein Gemisch aus Essigsäure und Wasser über eine Kolonne abdestilliert. Wenn keine Essigsäure mehr mit dem Wasser destilliert, kann auch das Lösemittel abdestilliert werden. Man kann jedoch das Produkt auch in dem Lösemittel umkristallisieren.

Geeignete Lösemittel haben vorzugsweise einen Siedepunkt im Bereich von 150 bis 270 °C. Verwendbar sind beispielsweise Kohlenwasserstoffe, Alkohole, Ester, Ether und Chlorkohlenwasserstoffe. Aus wirtschaftlichen Gründen werden marktgängige, preiswerte Produkte, wie z. B. Oxoalkohole, eingesetzt. Dabei ist beispielsweise auch 2-Ethylhexanol und dessen Acetat geeignet. Das Lösemittel kann während der Carbonsäureabspaltung oder zu einem früheren Zeitpunkt zugegeben werden.

Für die Umsetzung mit TAD werden Carbonsäure und Carbonsäureanhydrid vorzugsweise im Gewichtsverhältnis 10 : 1 bis 1 : 10 eingesetzt. Dabei stellt man besonders bevorzugt eine Gewichtsverhältnis von 5 : 1 bis 1 : 2 ein.

Vorzugsweise werden Carbonsäureanhydrid und TAD im Molverhältnis 4 : 1 bis 1 : 1 angewandt. Molverhältnisse von 1,5 : 1 bis 1,1 : 1 werden dabei besonders bevorzugt.

Die Menge an Wasser richtet sich nach der Dimensionierung der Apparatur und nach den angewandten Destillationsbedingungen. Es wird so lange Wasser oder Wasserdampf in das Reaktionsgemisch gegeben, bis das anfallende, wäßrige Kondensat keine oder nur noch geringe Mengen Carbonsäure enthält.

Das Lösemittel hat die Aufgabe, anfallendes Sublimat im oberen Teil des Reaktors zu lösen und in den Sumpf zu spülen. Das Lösemittel/TAD-Gewichtsverhältnis beträgt meist 5 : 1 bis 1 : 10 und in besonderen Fällen 1 : 1 bis 1 : 3.

Ein besonderer Vorteil des erfindungsgemäßen Verfahrens ist, daß nur flüssige, leicht entsorgbare Nebenprodukte anfallen, die zum größten Teil, so die abdestillierte Säure und das destillierte Lösemittel, zurückgewonnen und wieder eingesetzt werden können.

Ein weiterer Vorteil sind die schonenden Bedingungen und daß z. B. Acetyl-TAD in sehr hohen Ausbeuten von über 90 % und isomerenrein erhalten wird.

Der Stickstoff im Sechsring reagiert unter den angewandten Bedingungen nicht mit Acetanhydrid unter Bildung einer covalenten Bindung. Das ist überraschend, da in DE-A-38 00 987 ein Verfahren zur Herstellung von N-Acetyl-2,2,6,6-tetramethyl-4-piperidin beschrieben wird, bei dem das sogenannte Triacetonamin mit Acetanhydrid umgesetzt wird, wobei also der Stickstoff im Sechsring zur Reaktion gebracht wird.

Bei der Durchführung des erfindungsgemäßen Verfahrens wird beispielsweise eine Rührapparatur mit aufgesetzter Destillationskolonne verwendet. Man legt ein Gemisch aus Carbonsäure und Carbonsäureanhydrid vor und erwärmt auf die gewünschte Reaktionstemperatur, worauf dann TAD zudosiert wird. Nach der Reaktion werden überschüssiges Säureanhydrid und freie Carbonsäure abdestilliert, wobei eine flüssige, leicht rührbare Schmelze des Zwischenprodukts im Kolben verbleibt.

Dann werden Wasser oder Wasserdampf und gegebenenfalls ein Lösemittel zugegeben, wobei eine wäßrige Säure destilliert wird. Wenn die Säure vollständig oder weitgehend abdestilliert ist, liegt das gewünschte Produkt als Schmelze oder in dem zugesetzten Lösemittel gelöst vor. Im ersten Falle kann das Produkt direkt destilliert werden. Im zweiten Falle kann aufkonzentriert werden, worauf man das Produkt auskristallisieren lassen kann. Man kann aber auch zuerst das Lösemittel und dann das Produkt destillieren.

Die folgenden Beispiele sollen die Erfindung verdeutlichen.

### Beispiel 1

Man benutzt eine Glasapparatur, die aus einem Vierhalskolben mit Tropftrichter, Rührer, Thermometer, Destillationskolonne und Destillationsbrücke mit Vorlage besteht.

Man legt im Kolben vor:
- 225 g (2,14 Mol): Acetanhydrid (97%ig)
- 400 g: Essigsäure (99,8%ig)

Dann erwärmt man das Gemisch auf 90 °C und gibt innerhalb von 2 Stunden 313 g (2,0 Mol) TAD (99,8%ig) bei dieser Temperatur zu. Danach erwärmt man auf 100 °C und rührt eine Stunde nach.

Zur Bestimmung des Umsatzes wird eine Probe mit Methanol verdünnt, mit Natronlauge neutralisiert und gaschromatographisch analysiert. Der Gehalt an TAD ist kleiner als 0,1 %, d. h. TAD ist praktisch vollständig umgesetzt.

Die Temperatur wird dann von 100 auf 200 °C erhöht. Als Destillat erhält man 368 g reine Essigsäure.

Danach werden bei konstanter Temperatur von 200 °C 150 g 2-Ethylhexanol zügig zugegeben.

Anschließend wird mit der Zugabe von Wasser, 60 ml/h, begonnen. Die Menge an Destillat ist anfangs erheblich größer als die an eingesetztem Wasser, sie geht innerhalb von 2 Stunden von 65 ml pro halbe Stunde auf 35 ml pro halbe Stunde zurück. Nach 3 Stunden liegt die Säurezahl einer Destillatprobe, die zweiphasig ist, unter 3,0. Die Abspaltung der Essigsäure ist damit beendet.

Bei konstanter Sumpftemperatur von 200 °C wird nun durch Verminderung des Drucks auf zunächst 400 und innerhalb von 3 Stunden auf 30 mbar das 2-Ethylhexanol abdestilliert.

Es werden 127 g Destillat erhalten, das zu 38 % aus 2-Ethylhexanol und zu 60 % aus seinem Acetat besteht.

Das Sumpfprodukt ist reines, bräunlich gefärbtes Acetyl-TAD, dessen Gehalte an TAD, 2-Ethylhexanol und Acetat des 2-Ethylhexanols unter 0,1 % liegen. Es werden 364,5 g erhalten. Das entspricht einer Ausbeute von ca. 92 %, bezogen auf das eingesetzte TAD.

Dieses Produkt ist als Einsatzstoff zur Herstellung des N-Oxyls geeignet. Dazu wird es in Wasser suspendiert, mit Katalysator versetzt und mit Wasserstoffsuperoxid oxidiert. Nach erfolgtem Umsatz wird die Lösung abgekühlt und das N-Oxyl des Acetyl-TAD durch Filtration gewonnen.

### Beispiel 2

Man benutzt die im Beispiel 1 beschriebene Apparatur und setzt ein:
- 355 g (2,2 Mol): Buttersäureanhydrid (98%ig)
- 180 g: Buttersäure

Das Gemisch wird auf 80 °C erwärmt, worauf man innerhalb von 4 Stunden 313 g (2,0 Mol) TAD (98%ig) bei dieser Temperatur zugibt. Danach wird innerhalb von 1 Stunde auf 120 °C erwärmt und 1 Stunde bei dieser Temperatur gerührt. Danach ist der Umsatz an TAD vollständig.

Durch Erwärmen auf 220°C wird die Buttersäure abdestilliert. Danach wird als Lösemittel Tetralin bei 220 °C zugegeben und anschließend durch Zugabe von Wasser - wie beim Beispiel 1 beschrieben - Buttersäure abgespalten. Anschließend wird im Vakuum das zugesetzte Tetralin abdestilliert.

Das gewünschte Amid aus Buttersäure und TAD wird in einer Reinheit von 98 % und einer Ausbeute, bezogen auf eingesetztes TAD, von 94 % erhalten.

## Patentansprüche

1. Verfahren zur Herstellung von 4-Acylamino-2,2,6,6-tetramethylpiperidinen aus 4-Amino-2,2,6,6-tetramethylpiperidin und Säureanhydriden,
dadurch gekennzeichnet,
daß man die Umsetzung in Gegenwart von Carbonsäuren durchführt und die Carbonsäure aus dem zunächst gebildeten Carbonsäuresalz des 4-Acylamino-2,2,6,6-tetramethylpiperidins mit Hilfe von Wasser bei erhöhter Temperatur abspaltet.

2. Verfahren nach Anspruch 1,
dadurch gekennzeichnet,
daß man mit Säureanhydriden der Struktur 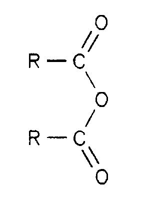 umsetzt, wobei R Alkyl mit 1 bis 7 C-Atomen oder Phenyl ist.

3. Verfahren nach Anspruch 1,
dadurch gekennzeichnet,
daß man die Umsetzung bei 80 bis 160 °C und die Abspaltung bei 170 bis 250 °C durchführt.

4. Verfahren nach Anspruch 3,
dadurch gekennzeichnet,
daß man die Umsetzung bei 90 bis 120 °C und die Abspaltung bei 190 bis 210 °C ausführt.

5. Verfahren nach Anspruch 1,
dadurch gekennzeichnet,
daß man die Carbonsäure in Gegenwart eines Lösemittels mit einem Siedepunkt von 150 bis 270 °C abspaltet.

6. Verfahren nach Anspruch 1,
dadurch gekennzeichnet,
daß Carbonsäure und Carbonsäureanhydrid im Gewichtsverhältnis von 10 : 1 bis 1 : 10 eingesetzt werden.

7. Verfahren nach Anspruch 1,
dadurch gekennzeichnet,
daß Carbonsäureanhydrid und 4-Amino-2,2,6,6-tetramethylpiperidin im Molverhältnis von 4 : 1 bis 1 : 1 eingesetzt werden.
